Europäisches Patentamt

(19) European Patent Office

Office européen des brevets

(11) Publication number

0 283 269
A2

# EUROPEAN PATENT APPLICATION

(12)

(21) Application number: 88302304.6

(22) Date of filing: 16.03.88

(51) Int. Cl.⁴: **A 61 B 17/14**
**B 27 B 19/00**

(30) Priority: 16.03.87 US 26038  29.09.87 US 105139

(43) Date of publication of application:
21.09.88 Bulletin 88/38

(84) Designated Contracting States: DE FR GB IT

(71) Applicant: ACE MEDICAL, INC.
14105 South Avalon Boulevard
Los Angeles California 90061-2691 (US)

(72) Inventor: Graham, Gregory S.
972 Waterbury Lane
Ventura California 93001 (US)

(74) Representative: Abrams, Michael John et al
HASELTINE LAKE & CO. Hazlitt House 28 Southampton
Buildings Chancery Lane
London WC2A 1AT (GB)

(54) **Microsurgical apparatus.**

(57) Microsurgical apparatus is disclosed which, in one aspect, comprises a support assembly (220) constructed and adapted to support a functional element (300), characterised in that the tool comprises a generally cylindrical hand grip member (232), a pivot (250) supported for pivotal motion inside the hand grip member, and a support for said functional element comprising a generally cylindrical member (260) and retaining means (269, 270, 271, 274, 276, 278, 280) for receiving and selectively securing and releasing said functional element (300). A support and drive mechanism is also provided, and comprises a rotary-to-lateral movement converting mechanism (150) including a concentric-eccentric rotor (152) and a way block (182) having a generally oval opening (192) which receives the eccentric shaft (158) of said rotor (152) so that rotary motion of rotor (152) is converted to reciprocal lateral motion of block (182).

The invention also provides a microsurgery saw (300) comprising an elongate structure having a proximal end and a distal end of from about three to about 20 cm. in length and a diameter of from about three to about 10 mm, a cutting tool (308) formed proximate the distal end of the elongate structure generally perpendicular to the longitudinal axis thereof, and engaging and retaining means (304, 306) formed proximate the proximal end of the elongate structure.

FIG. 3

## Description

### MICROSURGERY SAW DRIVE

This invention relates to microsurgical apparatus generally and more particularly but not exclusively, to apparatus for performing surgery on small bony and related structures such as, for example, the cervical vertebrae.

Microsurgical techniques are often very important in correcting diseased or damaged bone or other structure without the trauma of normal surgical techniques. For example, fusion of vertebrae of the spinal column is indicated in many cases of diseased or damaged vertebrae. There are several well-known procedures for performing vertebrae fusions and the techniques are generally described in voluminous literature on the subject. These general procedures are of limited applicability in treating diseases or injuries to the cervical vertebrae. Cervical vertebrae are delicate, with small bodies, and a very large neural canal. Consequently, the available bone is limited and many procedures which may be quite satisfactory in treating thoracic and lumbar vertebrae, which are much larger, are not applicable in the treatment of cervical vertebrae. One could also cite other microsurgical techniques, such as are used in certain joint surgical procedures.

According to one aspect of the present invention, there is provided a surgical tool which comprises a support assembly constructed and adapted to support a functional element, characterised in that the tool comprises a generally cylindrical hand grip member, a pivot supported for pivotal motion inside the hand grip member, and a support for said functional element comprising a generally cylindrical member and retaining means for receiving and selectively securing and releasing said functional element.

According to a second aspect of the present invention, there is provided a microsurgery tool support and drive mechanism constructed, dimensioned and configured so as to be adapted to drive an elongate microsurgery tool the proximal end of which is adapted to engage drive means forming part of said mechanism, characterised in that the mechanism comprises a rotary-to-lateral movement converting mechanism including a concentric-eccentric rotor having a main rotary portion, a concentric rotary portion and an eccentric rotary portion; a motor operably connected for driving the concentric-eccentric rotor; a way assembly mounted for reciprocal lateral motion perpendicular to the axis of the main rotary portion of the concentric-eccentric rotor, the way assembly comprising a body having formed therein a generally oval opening having opposed side walls, the rotor being constructed and disposed so that the eccentric rotary portion engages at alternating intervals the respective sides of said generally oval opening in the way body thereby moving the way body in reciprocal motion laterally; means in the way block for engaging the proximal end of the microsurgery tool and causing lateral movement thereof; and means for retaining the tool in engagement with the way block.

According to a third aspect of the present invention, there is provided a microsurgery saw comprising an elongate structure having a proximal end and a distal end of from about three to about 20 cm. in length and a diameter of from about three to about 10 mm, a cutting tool formed proximate the distal end of the elongate structure generally perpendicular to the longitudinal axis thereof, and engaging and retaining means formed proximate the proximal end of the elongate structure.

A feature of this invention is in the provision of a small, easily handled microsurgery saw drive for operating a delicate micro-saw in microsurgical procedures generally, e.g. in cervical vertebrae fusion procedures, and in other procedures where it is necessary or desirable to make small cuts in bone or rigid body structures. The drive of this invention is designed for use, most advantageously, with the microsurgery saw described herein, but is not so limited.

The present invention includes, in one aspect, a rotary-to-lateral motion converting assembly comprising a concentric-eccentric rotor constructed and arranged so as to form a central main rotor portion, a proximal concentric drive shaft portion and an eccentric distal drive shaft portion. A motor, which is conveniently an electric motor but may be fluid driven, or of any other type, is operably connected to the concentric-eccentric rotor for rotating the same. An eccentric drive shaft of the concentric-eccentric rotor engages a way block mounted for reciprocal, lateral travel. The eccentric drive shaft portion is disposed and constructed so as to engage the lateral way block so that, in response to drive power from the motor, the way block is driven reciprocally and laterally thereby converting the rotary motion of the motor to lateral motion of the way block.

The way block comprises, in a preferred but not required form, a generally rectangular main block portion having formed thereon ways which are constructed and disposed to movably support the way block for lateral movement in a plane generally perpendicular to the axes of the concentric-eccentric rotor. The way block is also preferably constructed and configured to define a generally oval aperture in the main block portion for receiving the eccentric shaft of the concentric-eccentric rotor. The aperture formed and configured to define side walls which are operably engaged alternately by the eccentric shaft portion thereof first on one wall for moving the way block in one direction laterally and then on the other wall for moving the block in the other direction laterally.

The mechanism is designed, in the preferred but not required embodiment, to drive a microsurgical saw, such as, for example, a cervical saw. In this embodiment, the way block also comprises saw engaging means for moving the proximal end of the microsurgery saw laterally. The mechanism may be designed such that the saw engaging means comprises a boss extending upwardly and distally from the main way block portion having formed therethrough a passage defined at the distal end by an oval opening having the major axis parallel to the plane in which the way block moves laterally and saw engaging structure accessible through said passage.

The way assembly preferably comprises a body having formed therein a generally oval opening having opposed walls and the rotor is preferably constructed and disposed so that the eccentric rotary portion engages at alternating intervals the respective sides of the generally oval opening in the way body for moving the way body in reciprocal motion laterally. The mechanism may be, but is not necessarily, constructed, dimensioned and configured to drive an elongate cervical saw having a proximal end adapted to engage drive means and a distal end adapted to cut bone, tissue and the like. In this form, the mechanism includes means for engaging the proximal end of the cervical saw and causing lateral movement thereof.

In the support assembly of this invention, the pivot, in the preferred form of this embodiment, consists of an omni-directional pivot ball and means supporting the ball for pivotal movement. The retaining means preferably, but not essentially, comprises at least one detent and one retaining ball in the detent. Generally, a pair of detents carrying a pair of balls is used, but one, two, three or more may be used if preferred.

In a more encompassing view, the invention provides a drive mechanism for supporting and driving an elongate, microsurgery tool, e.g. a generally cylindrical microsurgical saw for performing cervical and other microsurgical procedures. A microsurgery tool support assembly constructed and adapted to support an elongate generally cylindrical microsurgery tool, e.g. saw having cutting means at the distal end and means at the proximal end for engaging the way block, may be positioned adjacent the way block. The microsurgery support assembly preferably comprises a generally cylindrical hand grip member, a pivot supported for pivotal motion inside the hand grip member, and a microsurgery saw blade support comprising a generally cylindrical member and retaining means for receiving and selectively securing and releasing the microsurgery saw. The way block comprises saw engaging means for moving the proximal end of the microsurgery saw laterally, which may include a saw engaging structure comprising a pin for engaging in a slot in the proximal end of the microsurgery saw.

The invention is, thus, in one form a microsurgery saw support and drive mechanism so constructed, dimensioned and configured as to be adapted to drive an elongate microsurgery saw having a proximal end adapted to engage drive means and a distal end adopted to cut bone, tissue and the like, comprising rotary-to-lateral movement converting mechanism comprising a concentric-eccentric rotor forming a main rotary portion, a concentric rotary portion and an eccentric rotary portion, a motor operably connected for driving the concentric-eccentric rotor, means mounting the concentric-eccentric for rotation on the main rotary portion, a way assembly mounted for reciprocal lateral motion perpendicular to the axis of the main rotary portion of the concentric-eccentric rotor, the way assembly comprising a body having formed therein a generally oval opening having opposed side walls, the rotor being so constructed and disposed that the eccentric rotary portion engages at alternating intervals the respective sides of a generally oval opening in the way body for moving the way body in reciprocal motion laterally, a microsurgery saw support assembly constructed and adapted to support an elongate microsurgery saw cutting means at the distal end and means at the proximal end for engaging the way block, and means on the way block for engaging the proximal end of the microsurgery saw and causing lateral movement thereof. The microsurgery saw support assembly may comprise an elongate hand grip member, a pivot supported for pivotal motion inside the hand grip member, and a microsurgery saw blade support comprising an elongate saw receiving member for receiving therein the microsurgery saw and retaining means for receiving and selectively securing and releasing the microsurgery saw.

In a microsurgery saw of this invention, the engaging means may comprise a slot transversely formed in the proximal end of the elongate structure, and the retaining means may comprise an annular groove circumscribing the elongate structure proximate the proximal end thereof. The elongate structure preferably is generally cylindrical along a major portion of the length thereof.

In yet another aspect, the invention provides the combination of drive means for producing rotary motion, rotary-to-lateral converting means operatively driven by the drive means, saw support means for pivotally supporting a microsurgery saw operatively connected to the converting means for transmitting lateral drive motion to the microsurgery saw, drive casing means surrounding and positioning the drive means and saw support casing means surrounding and positioning the saw support means. In a preferred embodiment, the drive casing means and the saw support casing means are generally cylindrical, each having a generally central axis, the casing means being secured to each other such that the angle between the axes of the casing means is about fifteen degrees, the exact angle not being extremely critical, angles of from about ten to about 30 degrees being acceptable, though the fifteen degree angle is strongly preferred.

For a better understanding of the invention, and to illustrate how the same may be put into effect, reference will now be made, by way of example, to the accompanying drawings in which only one exemplary embodiment is depicted, it being expressly pointed out that only the currently preferred mode is described though many variations are possible within the scope of the invention. In the drawings:

Figure 1 is a side view of a cervical saw drive in accordance with this invention;

Figure 2 is a side view in cut-away and cross section of the main cylindrical part of the drive and the rotary-to-lateral motion conversion mechanism of the present invention;

Figure 3 is a continuation, to the right as viewed, of the mechanism of Figure 2, some components being duplicated for clarity of presentation, showing a cervical saw blade support and holder portion in accordance with this invention, the cervical saw being shown in position for use;

Figures 4A and 4B show the retaining and release positions of the cervical saw blade support and holder with the saw positioned therein;

Figure 5 is a partial view, in cross section showing details of the ball-detent locking mechanism for the support for the cervical saw;

Figure 6 is a partial, exploded view, partially schematic, of a rotary-to-lateral motion conversion mechanism in accordance with the present invention;

Figure 7 is an end view, taken along Lines 7--7 of Figure 6, showing the concentric-eccentric rotor of the rotary-to-lateral motion conversion mechanism;

Figure 8 is a face view, taken along Lines 8--8 of Figure 6, of the way block assembly of this invention;

Figure 9 is a schematic view showing the way block and the means for laterally driving the same and depicting the lateral movement of the same;

Figure 10 is a side view of a microsurgery saw of the type which may be used with this invention; and

Figure 11 is an enlarged, perspective view of the proximal end portion of the saw depicted in Figure 10.

In describing the following embodiment, which is currently the preferred embodiment, it is to be clearly understood that the structures and interrelationships are described in detail as an example of the invention and not as the only form of the invention; indeed, many forms are possible for the invention and many substitutions and variations can be made without departing from the scope of the invention. For example, the casing for the cervical saw is convenient but of no criticality insofar as the invention is concerned. Likewise, any light-weight motor can be used. For example, fluid turbines may be used in lieu of electric motors, such as, by way of example only, the fluid turbine motors commonly used in dental tools. Except as specifically claimed and limited in the claims, the interconnections and configurations are merely exemplary of a great variety of variations which can be made without departing from the invention.

An overall description of the invention will be provided first, with details of certain mechanisms and relationships being described thereafter.

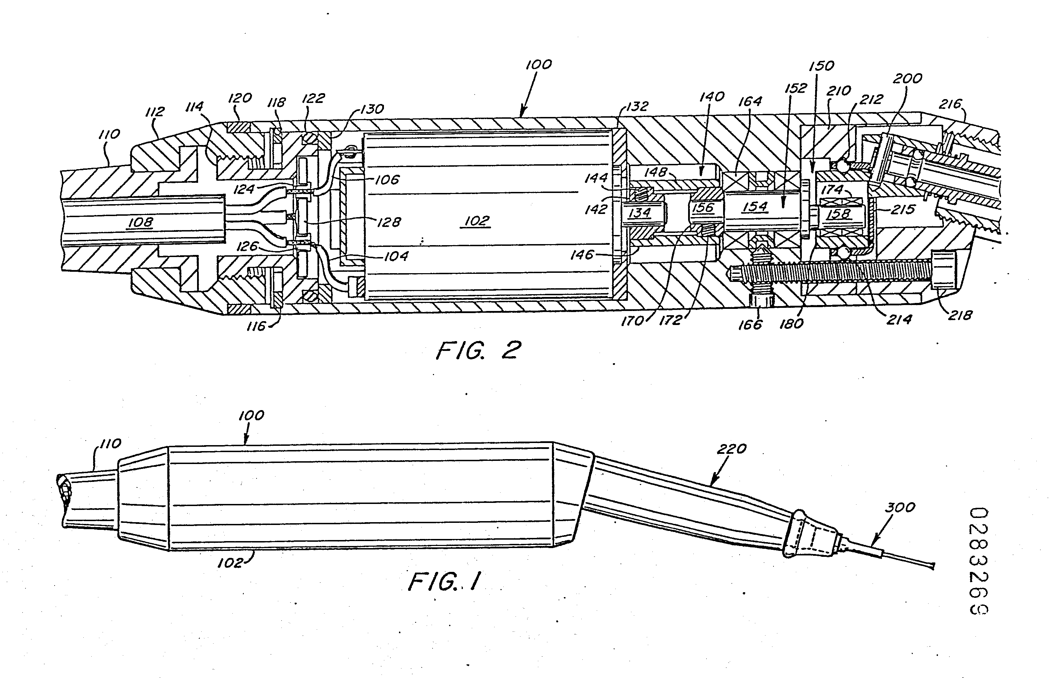

The cervical saw drive, generally shown in Figure 1 and shown in greater detail in Figure 2, comprises a casing 100 which is a generally elongate cylinder having external structure for providing gripping surfaces, to permit the surgeon to grip the drive during use, and internal structure for receiving and positioning drive motor 102 and the interconnected drive mechanisms.

The power for driving the saw, in this example, is an electric motor 102 which receives electrical energy from wires 104 and 106 of cord 108 which is provided with a plug and adapted to plug into any compatible electric energy source. The cord 108 is surrounded by a strain relief sleeve 110 the proximal end of which is attached to the casing 100 by an internally threaded retaining sleeve 112 which, in turn, is held in the casing 100 by externally threaded sleeve 114, which is secured by retaining ring 116 which seats in groove 118 which is formed in the interior of casing 100. Ring 120 surrounds the proximal, internally threaded end of retaining sleeve 112 assuring a tight, substantially moisture-proof seal between the threads of the retaining sleeve 112 and the threads of externally threaded sleeve 114. The proximal end of the externally threaded sleeve 114 forms an annular land upon which an O-ring 122 rests, the O-ring 122 forming a seal between the interior surface of casing 100 and sleeve 114. The strain relief covering 110 is formed of rubber or other resilient polymer to permit the cord to bend gradually and prevent sharp bends which would unduly strain the cord. The internally threaded sleeve 112, the strain relief covering 110, the externally threaded sleeve 114 and the O-ring 122 form a substantially water tight seal for preventing moisture from entering the casing.

The power providing conduits 104 and 106 extend through grommets 124 and 126 in passages through insulator disc 128 and are attached to the input terminals of motor 102 according to conventional electrical wiring procedures. The insulator disk 128 is disposed against a shoulder formed in the proximal portion of the externally threaded sleeve 114 and is spaced from the distal end of motor 102 by a spacer ring 130. The proximal end of the motor 102 is held in position and sealed by a retaining and sealing ring 132. The spacer rings 130 and 132 secure the motor 102 in insulated, fixed position in the casing 100. The combination of these seals prevents moisture from entering the portion of the casing occupied by the electrical connections and motor under most environmental conditions, and mounts the motor for operation in the casing, but does not necessarily seal the motor sufficiently to permit immersion of the drive.

The rotationally driven shaft 134 of the motor 102 is operationally connected to a drive coupling mechanism indicated generally at 140 comprising sleeve 142 secured to the shaft 134 by a set screw 144 and engages internally splined or geared coupling sleeve 148.

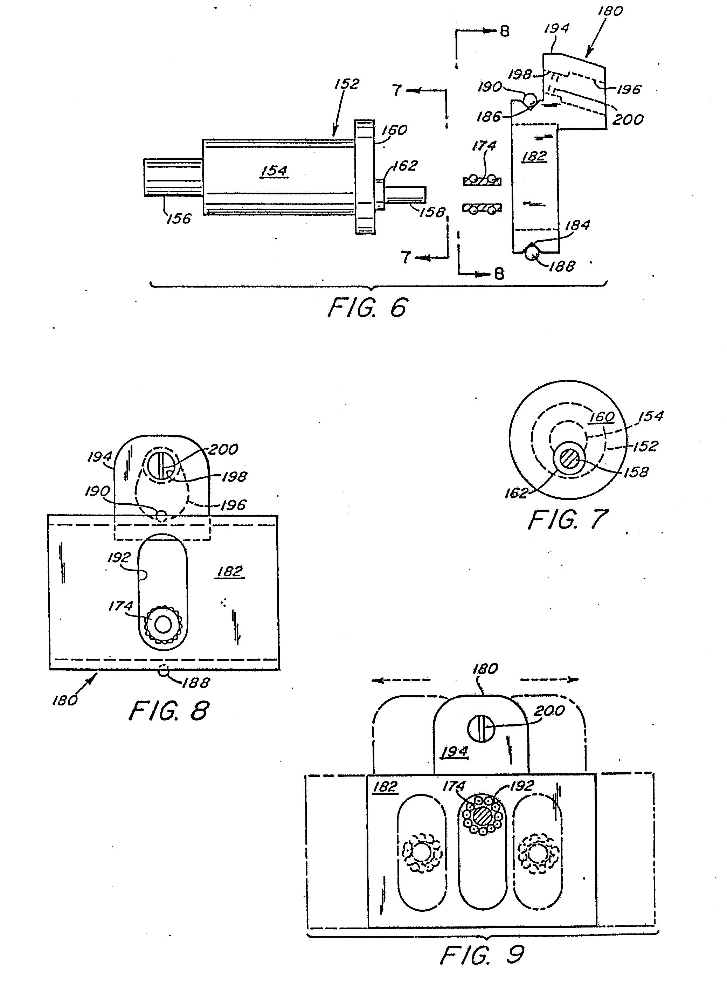

A rotary-to-lateral motion converting assembly indicated generally at 150 in Figure 2, and shown in greater, somewhat schematic, detail in Figures 6 - 8 inclusive, to which reference is now made, is disposed in the proximal portion of the sleeve 100. A concentric-eccentric rotor 152 comprising a central main rotor portion 154, a proximal concentric drive shaft portion 156 and an eccentric distal drive shaft portion 158, and which may conveniently include a flange 160 on the main rotor portion and a shoulder 162 on the eccentric drive shaft, is mounted for rotation in the sleeve by a double-ball bearing assembly 164 conveniently held in place by set screw 166. The distal drive is operably connected to the motor 102 by means of an external spline gear 170 mounted by a set screw 172 to the concentric drive shaft portion 156 of the concentric-eccentric rotor 152 and in internal spline gear sleeve 148 which engages the corresponding spline gear 142 on the output shaft 134 of the motor 102. The concentric-eccentric rotor 152 is thus rotatably driven by the motor 102.

The eccentric drive shaft 158 carries a ball bearing assembly 174 which interacts with lateral way block 180 to convert the rotary motion of the concentric-eccentric rotor to lateral motion of the way block in a manner which will now described.

The way block 180 comprises a generally rectangular main block portion 182 (though the configuration is not critical) provided, in the preferred embodiment, with V-shaped ways 184 and 186 which are adapted to engage balls 188 and 190 which movably support the bottom and the top, respectively, of the way block 180 for lateral movement in a plane perpendicular to the axes of the concentric-eccentric rotor 152. A generally oval aperture 192 is formed in the main block portion 182 having, in the preferred form, generally straight sides and generally arcuate ends, though the shape is not critical so long as it is generally oval. As the concentric-eccentric rotor 152 is caused to rotate by operation of the motor 102, the eccentric shaft portion thereof 158, through bearing 174, engages the side walls of the aperture 192, first one side moving the way block 180 to the right then the other side moving the way block to the left as shown in Figure 9.

The way block 180 also comprises, in this preferred embodiment, saw engaging means which function to move the proximal end of the cervical saw laterally, as will be described. The saw engaging means comprises a boss 194 extending upwardly and distally from the main way block portion 182 having formed therethrough a stepped passage defined at the distal end by an oval opening 196 having its major axis parallel to the plane in which the way block moves laterally and having a smaller opening 198 which is traversed by a saw engaging pin 200 (see Fig. 2). It is pointed out, however, that any means of engaging and moving the proximal end of the cervical saw laterally with the lateral movement of the way block can be used and that the use of the apertured boss is simply one convenient means for accomplishing this function. It will also be noted that the aperture extends through the boss only because this is a convenient and inexpensive way to machine the saw engaging means and that the smaller opening 198 need not extend through the boss.

To review, the rotary-to-lateral movement converting mechanism 150 of this invention comprises concentric-eccentric rotor 152 driven by the motor and mounted on main rotary portion 154 coaxial with or on a parallel axis with the axis of the motor 102 (though this axial relationship is not critical) having an eccentric rotary portion 158 which lies on an axis parallel to the main axis (the axis through the main rotary portion 154) but offset therefrom which engages at alternating intervals the sides of a generally oval vertically orientated opening 192 in the main portion of the way block 180. The major axis of the oval opening 192 is perpendicular to both the direction of lateral movement and the main axis of the concentric-eccentric rotor 152, thus the eccentric rotary portion 158 moves the way block 180 laterally and not vertically, as the eccentric rotary portion 158 does not engage the ends of the oval opening 192. The way block 180 includes means for engaging the proximal end of the cervical saw and causing lateral movement thereof. In the foregoing summary, structures which are necessary for long-term reliable operation but not important to the concept of the rotary-to-lateral movement converting mechanism 150 have not been mentioned for the sake of simplicity.

As seen in Fig. 2, way mounting ring 210 has formed therein a pair of ways 212 and 214 which receive balls 188 and 190, respectively. The balls 188 and 190, held in position by cage 215, support way block 180 for reciprocal lateral movement. Thus, the mounting ring 210 along with the balls 188 and 190 form support means for and permit reciprocal lateral movement of the way block 180. Way mounting ring 210 and a proximal end closure block 216 are held in place closing the proximal end of the casing, and sealing to the body by "O" ring 217, by retaining screw 218 which is screwed into a matching threaded aperture in the casing. These last described mechanisms are not critical as to shape or configuration and any convenient support for the way block 180 and closure for the sleeve may be used.

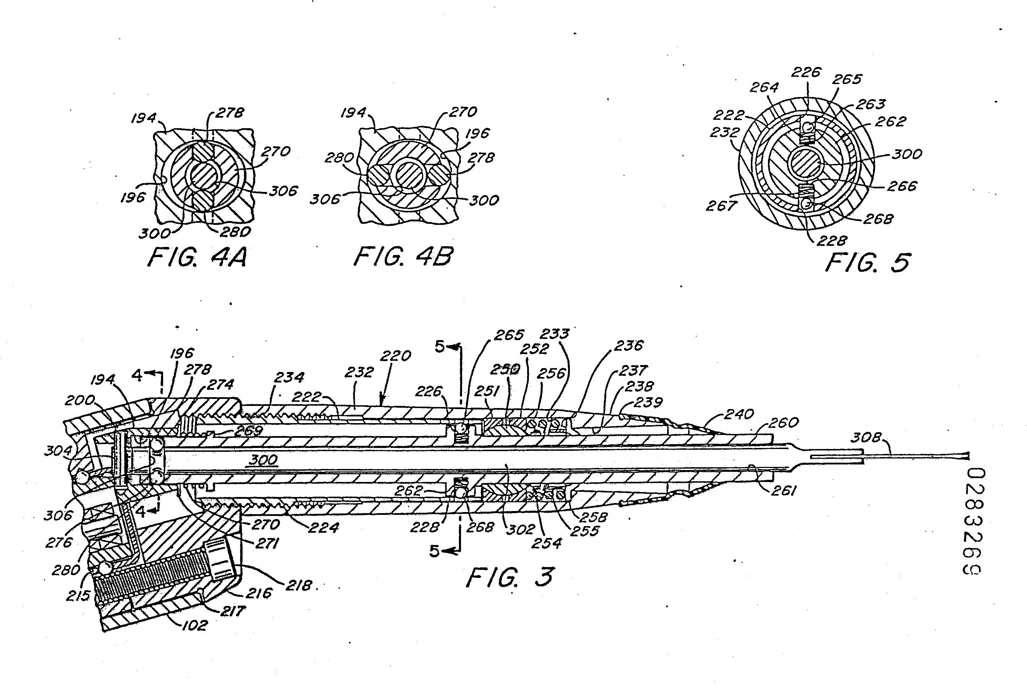

While the mechanism thus far described can be used for many purposes, it is used, in this preferred embodiment, in connection with a cervical saw and cervical saw support assembly. The cervical saw support assembly 220, best shown in Figures 3, 4 and 5, to which reference is now made, comprises cylindrical guide sleeve 222, which at its proximal end is threaded as shown at 224, is secured into the closure 216 and extends therefrom at an angle of about 15 degrees.

An outer sleeve 232 has a larger interior cylindrical portion 233 which is threaded at the proximal end, as shown at 234, and terminates in a shoulder 236 forming a smaller interior cylindrical portion 237 near the distal end, where the outer surface 238 is generally frustroconical and is configured and constructed to be easily held by the surgeon. An annular groove 239 is also formed near the distal end of the sleeve 232 to receive a sealing lip of a disposable sealing boot 240 which seals against the cylinder 232 and against the blade guide and pivotal holder 260.

An omni-directional pivot ball 250 is mounted in the sleeve portions 233 of the outer sleeve 232 by bearings 251 and 252, and is loaded (spring biased) inwardly by a spring guide 254 at the proximal end of the spring and another spring guide 255 at the distal end of the spring which hold the spring 256 in the cylinder portion 233, the spring guide bearing against an annular seal 257 and the shoulder 246 formed in the sleeve 232, as previously described. The seal 257 seals between the inerior of the sleeve 232 and the blade guide 260 which, except as described hereinafter, has a cylindrical exterior configuration.

The blade guide 260 has an interior cylindrical configuration as shown at 261.

Between the proximal and distal ends of the blade guide 260 an annular sleeve 262, or a pair of bosses, extends outwardly from the cylindrical body of the guide in which are formed two recesses. Recess 263, which receives a bias spring 264 and ball 265, and recess 266, which recieves bias spring 267 and ball 268, extend diametrically oppositely from the body of the guide. The balls 265 and 268 are received in slots 226 and 228 of the sleeve 222 serving to preload the blade guide, and the blade, to the centre.

Nearer the proximal end of the blade guide another sleeve 269 is formed which cooperates with spring 270 and wear washer 271 to preload the blade guide against the end of the boss 194.

Near the proximal end of the blade guide, the walls thereof define two detents 274 and 276 for balls 278 and 280. As will be described more specifically hereinafter, the blade 300 comprises a cylindrical portion 302 having a slot 304 for receiving the pin 200 which drives the blade reciprocally laterally and a detent groove 306 into which the balls 278 and 280 are received, securing the blade in place.

In use, a cervical saw 300 is received and retained in the cervical saw blade support and holder 260 by the coaction of the annular recess 306 formed therein which forms a detent for the balls 278 and 280. The cervical saw comprises an elongate cylindrical shaft 302 having formed in the proximal end thereof engaging means conveniently in the form of a slot 304 perpendicular of the axis of the shaft 302 for engaging the pin 200 which moves reciprocally as part of the way block 180 and, adjacent thereto, an annular groove 306 which is formed in the cylindrical portion 302 for receiving the retaining balls 278 and 280; and a distal cutter tip 308 which may be straight or bent or curved and may have any type of teeth, cutting edges, serrations, etc. thereon, as desired.

As shown in Figures 4A and 4B, the aperture 196 is not round but is rather oval or elliptical. As the end of the blade 300 is inserted into the drive, the end 304 engages the drive pin 200 and the blade guide is rotated 90 degrees at which position the balls 265 and 268 engage in the slots 226 and 228 and lock the blade guide, and the blade, into the instrument and give a distinct "click" assuring the surgeon that the blade is properly secured. The secured position is shown in Figures 3 and 4A. By rotating the blade guide another 90 degrees or back 90 degrees the balls 278 and 280 are disposed along the major diameter of the oval aperture as shown in Figure 4B, permitting the blade and blade guide to be removed and a new blade inserted in the same orientation and locked by turning 90 degrees as previously described.

The structure, function and mode of operation of the rotary-to-lateral motion conversion mechanism 150 has been described. The mechanism thus described and the drive, support and related mechanisms act to drive the distal cutting tip of the cervical saw 300 laterally in a reciprocating arcuate path, though the radius of the arc of movement is so large that arcuate shape of the cut can largely be disregarded.

In operation, the way block 180 is moved laterally by the motor 102 through the rotary-to-lateral motion conversion mechanism 150 as described, and thus moves the proximal end of the cervical saw 300 laterally, the slotted proximal end operatively engaging the pin 200 as the way block receiprocates laterally back and forth. The cervical saw 300 is caused thereby to pivot on the omni-directional pivot ball 250 thus causing the distal cutting tip to reciprocate laterally in a plane substantially perpendicular to the plane in which the way block 180 reciprocates. Lateral reciprocation of the cutter tip 308 is operative to cut or saw bone or other body structure.

The apparatus of the present invention can be made of many materials and in many forms, the form illustrated and described above being merely exemplary.

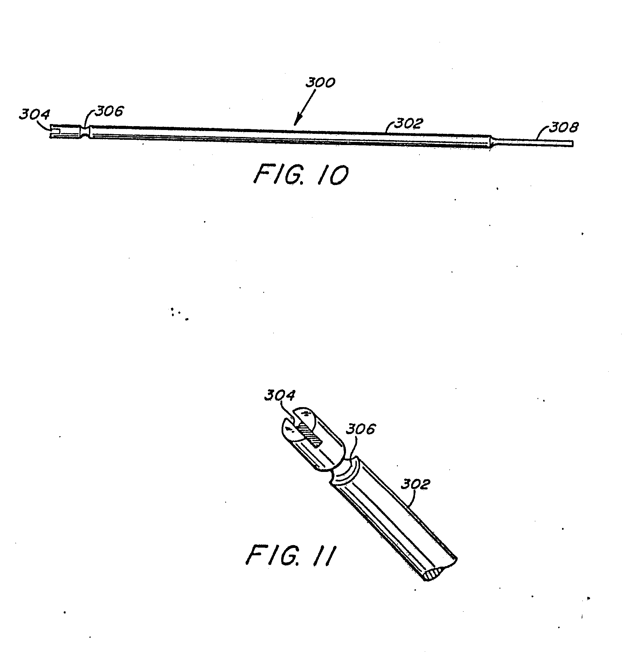

In a typical, preferred form, the microsurgery saw is an elongate, generally cylindrical saw having a diameter of about 4 mm, but the diameter may range from about 2 or 3 mm to as much as 6 or 8 mm, and having a length about 3 or 4 up to about 15 or 20 cm. The cutting edge of the saw is typically about 6 to 8 mm in width, but may be from 2 to 3 mm up to about 25 mm or more, typically. As shown in Figures 10 and 11, the microsurgery saw 300 preferably comprises an elongate cylindrical shaft portion 302 having formed at the proximal end drive engaging means such as slot 304 and retaining means such as the annular groove 306; and having at the distal end a cutting tool portion 308.

This invention is useful in orthopaedic and other surgical procedures.

## Claims

1. A surgical tool which comprises a support assembly (220) constructed and adapted to support a functional element (300), characterised in that the tool comprises a generally cylindrical hand grip member (232), a pivot (250) supported for pivotal motion inside the hand grip member, and a support for said functional element comprising a generally cylindrical member (260) and retaining means (269, 270, 271, 274, 276, 278, 280) for receiving and selectively securing and releasing said functional element (300).

2. A surgical tool as claimed in claim 1, wherein said pivot comprises an omni-directional pivot ball (250) and means (251, 252) supporting the ball for pivotal movement.

3. A surgical tool as claimed in claim 1 or 2, which further comprises a functional element in the form of an elongate tool (300) having engaging and retaining means (304, 306) formed proximate the proximal end thereof.

4. A surgical tool as claimed in claim 3, wherein said functional element is an elongate generally cylindrical cervical saw having cutting means (308) at the distal end thereof.

5. A surgical tool as claimed in claim 4, wherein said functional element is a cervical saw (300) which comprises an elongate structure of from about three to about 20 cm. in length and with a diameter of from about three to about 10 mm, and wherein said cutting tool (308) formed proximate the distal end of the elongate structure is generally perpendicular to the longitudinal axis thereof.

6. A surgical tool as claimed in claim 3, 4 or 5, wherein said retaining means comprises at least one detent (306) and at least one one retaining ball (278) in the detent.

7. A microsurgery tool support and drive mechanism constructed, dimensioned and configured so as to be adapted to drive an elongate microsurgery tool the proximal end of which is adapted to engage drive means forming part of said mechanism, characterised in that the mechanism comprises a rotary-to-lateral movement converting mechanism (150) including a concentric-eccentric rotor (152) having a main rotary portion (154), a concentric rotary portion (156) and an eccentric rotary portion (158); a motor (162) operably connected for driving the concentric-eccentric rotor (152); a way assembly (180) mounted for reciprocal lateral motion perpendicular to the axis of the main rotary portion (154) of the concentric-eccentric rotor (152), the way assembly comprising a body (182) having formed therein a generally oval opening (192) having opposed side walls, the rotor (152) being constructed and disposed so that the eccentric rotary portion (158) engages at alternating intervals the respective sides of said generally oval opening (192) in the way body (182) thereby moving the way body in reciprocal motion laterally; means (194) in the way block (180) for engaging the proximal end of the microsurgery tool and causing lateral movement thereof; and means for retaining the tool (300) in engagement with the way block (182).

8. A mechanism as claimed in claim 7, which further comprises means mounting the concentric-eccentric (152) for rotation on the main rotary portion.

9. A mechanism as claimed in claim 7 or 8, which further comprises a microsurgery tool support assembly constructed and adapted to support an elongate microsurgery tool having means at its proximal end for engaging the way block (180).

10. A support and drive mechanism as claimed in claim 7, 8 or 9, wherein said means for retaining the microsurgery tool in engagement with said way block (182) comprises at least two spring biased ball detent mechanisms.

11. A mechanism as claimed in claim 7, 8, 9 or 10, wherein the microsurgery tool support assembly comprises: an elongate hand grip member (232), and a microsurgery tool support comprising an elongate tool receiving member (260) for receiving therein the microsurgery tool and retaining means for receiving and selectively securing and releasing the microsurgery tool (300).

12. A mechanism as claimed in claim 11, wherein the means for securing the tool receiving member comprises a pair of opposed ball detents, a first pair (184, 186) proximate the proximal end thereof engaging the way block and a second pair (226, 228) proximate the centre of the axial length of the receiving member engaging the hand grip member.

13. A microsurgery saw (300) comprising an elongate structure having a proximal end and a distal end, e.g. of from about three to about 20 cm. in length and a diameter of from about three to about 10 mm, a cutting tool (308) formed proximate the distal end of the elongate structure generally perpendicular to the longitudinal axis thereof, and engaging and retaining means (304, 306) formed proximate the proximal end of the elongate structure.

14. A microsurgery saw as claimed in claim 13, wherein the engaging means comprises a slot (304) transversely formed in the proximal end of the elongate structure.

15. A microsurgery saw as claimed in claim 13 or 14, wherein the retaining means comprises an annular groove (306) circumscribing the elongate structure proximate the proximal end thereof.

16. A microsurgery saw as claimed in claim 13, 14 or 15, wherein the elongate structure is generally cylindrical along a major portion of the length thereof.

FIG. 2

FIG. 1

0283269

FIG. 5

FIG. 4A

FIG. 4B

FIG. 3

0283269

FIG. 6

FIG. 7

FIG. 8

FIG. 9

0283269

FIG. 10

FIG. 11